## Europäisches Patentamt
## European Patent Office
## Office européen des brevets

(11) Publication number: **0 072 251**
**B1**

(12) **EUROPEAN PATENT SPECIFICATION**

(45) Date of publication of patent specification: **09.05.90**

(21) Application number: **82304224.7**

(22) Date of filing: **10.08.82**

(51) Int. Cl.⁵: **A 61 L 15/16** // A61L15/22, A61L15/24, A61L15/26, A61L15/28, A61L15/44

(54) Improved bandage containing a medicament.

(30) Priority: **10.08.81 US 291611**

(43) Date of publication of application:
**16.02.83 Bulletin 83/07**

(45) Publication of the grant of the patent:
**09.05.90 Bulletin 90/19**

(84) Designated Contracting States:
**DE FR GB**

(56) References cited:
EP-A-0 013 606      FR-A-2 421 610
EP-A-0 017 401      US-A-4 291 015
WO-A-80/01138      US-A-4 306 551
FR-A-2 392 677      US-A-4 307 717

CHEMICAL ABSTRACTS, vol. 87, no. 18, 31st
October 1977, page 330, no. 141303j, Columbus,
Ohio, USA
CHEMICAL ABSTRACTS

(73) Proprietor: **LecTec Corporation**
**10215 Crosstown Circle**
**Eden Prairie Minnesota 55344 (US)**

(72) Inventor: **Hymes, Alan C.**
**3828 Wilmott Road**
**Hopkins Minnesota 55343 (US)**
Inventor: **Ong, Lincoln T.**
**5940 Fairwood Drive**
**Minnetonka Minnesota 55343 (US)**
Inventor: **Persons, Garry R.**
**5505 Highland Road**
**Edina Minnesota 55436 (US)**

(74) Representative: **Williams, John Francis et al**
**J.F. Williams & Co 34 Tavistock Street**
**London WC2E 7PB (GB)**

## Description

This invention relates to an improved bandage which contains a medicament that is topically released into the skin.

Attempts have been made to develop bandages which are self-adhesive, absorbent, and sterile. For example, U.S. Patent No. 3,339,546 discloses a self-adhesive bandage which is adapted to adhere to moist surfaces such as the moist mucosa of the oral cavity. However, one of the essential materials of this self-adhesive bandage is an adhesive gum, preferably polyisobutylene, which is hydrophobic. Similarly, U.S. Patent Nos. i.e. US—A—3,598,122 and 3,598,123 disclose bandages which contain drugs that are continually released from an adhesive layer. These bandages are formed of layered materials which have drugs encapsulated in the adhesive layer. Even though the bandages disclosed in these prior art patents are said to be self-adhesive and are satisfactory vehicles for drugs, specific process steps are required for encapsulating or stratifying the drugs.

EP—A—2-0 013 606 discloses a polymeric diffusion matrix comprising glycerol, polyvinylalcohol, a water-soluble polymer having hydration sites which, in combination with the remaining ingredients, yields a matrix capable of sustained release of a drug dispersed therein, and water. The diffusion matrix may be embedded in a cavity in a backing member having a pressure-sensitive adhesive layer surrounding the matrix.

It is a general object of this invention to provide a self-adhesive, novel bandage in which a medicament is molecularly dispersed for release to the affected area.

The present invention provides a flexible adhesive bandage for delivering a medicament to a patient, which bandage comprises a flexible backing element and a substrate, which substrate contains a medicament, the bandage having an adhesive surface for contact with and adhesion to a patient's skin, characterised in that the backing element is selected from cotton, paper, synthetic fabrics, and plastics materials, the substrate is liquid-absorbent and self-adhesive and comprises a homogeneous, hydrophilic, stable matrix sufficizntly pliant to conform to the shape of the body contours, the matrix comprising from 15% to 50% by weight, based on the total weight of the matrix, of a solid phase formed from a hydrophilic high-molecular weight polysaccharide and/or synthetic gum selected from polyacrylic acid, polyacrylamide, and their cogeners, vinyl acetate ethylene copolymer, vinyl acetate dioctyl maleate copolymer, natural gums, and starch-g-poly® (acrylamide-co-sodium acrylate), and from 30% to 70% by weight, based on the total weight of the matrix, of a liquid phase consisting of a solution or emulsion selected from carbohydrates, proteins, and polyhydric alcohols, the matrix containing a medicament uniformly molecularly dispersed throughout the substrate, which medicament is selected from vaso dilators, antibacterial agents, antiseptic agents, antifungal agents, antihistamine agents, anti-inflammatory agents, antipruretic agents, hormonal agents, keratolytic agents, skin protective agents, and rubefacient agents.

The bandage of the invention comprises a flexible backing element and a self-adhesive substrate which becomes increasingly tacky in the presence of moisture and which absorbs liquid and releases the medicament to the affected area while remaining *dimensionally stable*.

These and other objects and advantaeges of this invention will more fully appear from the following description made in connection with the accompanying drawings, wherein like reference characters refer to the same or similar parts throughout the several views.

Figures of the drawings
Fig. 1 is a perspective view illustrating the novel bandage applied to the arm of a patient.
Fig. 2 is a perspective view of a bandage illustrated in Fig. 1.
Fig. 3 is a perspective view of a bandage used as a surgical dressing.
Fig. 4 is a cross-sectional view taken approximately along line 4—4 of Fig. 2 and looking in the direction of the arrows.
Fig. 5 is a modified form of the bandage.

Description of the preferred embodiment
The bandage of the present invention has adhesive properties for maintaining contact with the skin, as well as possessing a certain amount of elasticity for movement with the skin. The bandage is intended to be easily handled and is non-irritating to the patient.

Referring now to the drawings, it will be seen that the bandage of the present invention is thereshown. The bandage, designated generally by the reference numeral 10, includes a backing member 11 and a self-adhesive substrate 12 which is secured to one end surface of the backing. The backing element 11 and the substrate 12 are both illustrated as rectangular sheets of material of uniform thickness. It is pointed out that the bandage 10 is intended to be regular in shape but may have any other configuration although the rectangular shape is preferred. In use, the bandage is applied with the substrate 12 in direct contact with the skin to cover a non-surgical wound, a surgical wound, or burned tissue. In Fig. 3, the embodiment illustrated therein is a surgical dressing and will be applied to the patient to cover a surgical wound.

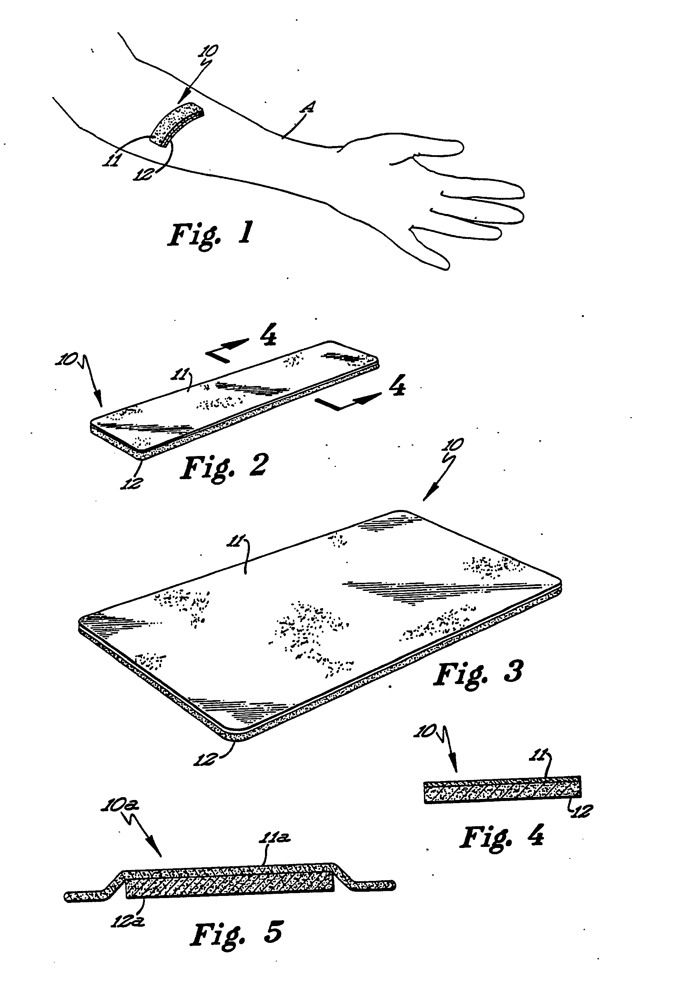

Referring now to Fig. 5, it will be seen that a different embodiment of the bandage, designated generally by the reference numeral 10a, is thereshown. The bandage includes a pressure-sensitive adhesive element 11a which serves as the backing element and also serves as the means for securing the bandage to the surface of the skin. The pressure-sensitive adhesive element 11a may be formed of any of the materials used in commercially available adhesive elements such as a foam-tape adhesive element. It will be appreciated that most of the commercially available adhesive elements maintain an excellent bond with the skin and are not irritable with respect to the skin.

Primary to the unique structure of the bandage is the hydrophilic adhesive properties of the substrate which enhance the adhesion thereof to the skin. The substrate not only absorbs moisture, making it ideal for use as a surgical dressing, but the substrate becomes tackier as it absorbs moisture.

The substrate 12 may be formed from naturally occurring materials such as gum karaya, guar gum, gum acacia, locust bean gum and other polysaccharides. The substrate may also be formed from synthetic polymers such as polyacrylamide and its cogeners, such as methylene-Bis-acrylamide, polyacrylic acid molecular weights 250,000, 450,000, 1,000,000, and 4,000,000, and polyacrylamide solid under such trademarks as Reten by Hercules Company. When monomers such as acrylic acid or acrylamide are polymerized, it is necessary to use activators. Activators, which are used during polymerization, may include ferrous sulfate, sodium metabilsulfite, potassium persulfate.

The synthetic polymers and/or natural gums and other polysaccharides constitute the solid phase of the matrix. The liquid phase of the matrix preferably consists of polyhydric alcohols such as glycerol or propylene glycol. Solutions or emulsions of saccharides and/or polysaccharides and/or proteins may be used in forming the matrix. Alternatively, a combination of a solution or emulsion of polysaccharides, saccharides, or proteins may be used in the liquid phase of the matrix.

The substrate 12 which is a stable matrix includes a *solid phase* comprising a synthetic polymer mixture, a karaya matrix, or a matrix or karaya and synthetic polymer. The solids of the matrix comprise 15% to 50% by weight of the matrix 11. The substrate may be sterilized. If the substrate is sterilized, the combination of the mixture is subjected to irradiation (usually gamma rays) of 2.5 mega rads for sterilization. Heretofore, this magnitude of irradiation to mixtures of polysaccharides, such as karaya with a polyhydric alcohol, preferably glycerol, would cause the matrix to lose dimensional stability with only slight pressure and/or water absorption. This causes the matrix to become so tacky that it is not manageable as a surgical bandage. Further, if this irradiated karaya is used as a sterile pad to seal drainage as noted in the US—A—3,640,741, it may readily break down to a gelatinous substance which may run and break the seal.

The *liquid phase* of the matrix, such as hydric alcohol, comprises 30% to 70% by weight of the matrix.

The bandage also includes a suitable backing member which may include cotton fabric, woven or standard paper, synthehic fabrics, and/or plastics. Suitable synthetic fabrics may include nylon or polyester while a suitable plastic backing may include mylar or saran. When the bandage 10 is used as a surgical dressing, the backing element comprises a pervious material such as cotton fabric to permit diffusion of the absorbed liquid into the air.

The substrate 12 also contains a medicinal substance for release to the surface to which the bandage is applied. The medicinal substance is molecularly dipersed in the matrix rather than being encapsulated as in the prior art. The medicinal substance may include an antibacterial, antiseptic, or antifunginal agents such as boric acid, bacitracin, acriflavin, formaldehyde, gential violet, mercuric sulfide, mercurochrome, neomycin, and iodine. Nitroglycerine may be used as a coronary vaso dilater agent and hydrocortisone may be used as an anti-inflammatory agent. Suitable antipruretic agents include benzoin, calamine, camphor, menthol, phenol, and sulfur. The substrate may also include fragrances such as cinnamon oil, fir needle oil, lemon oil, peppermint oil, and spearmint. Suitable healing agents include allantoin, Peruvian balsam, Vitamin A, and Vitamin E. Hormonal agents may include estrogen, progesterone, and testosterone. Protective agents may include benzoin, charcoal, talc, and zinc oxide. Salicylic acid is a suitable keratolytic agent and methyl salicylate is a suitable rubefacient. An exemplary antihistamine is chlorpheniramine.

The bandage also includes a suitable backing member which may include cotton fabric, woven or standard paper, synthetic fabrics, and plastics. Suitable synthetic fabric may include nylon or polyester while a suitable plastic backing may include mylar or saran.

When karaya or other natural gums are used in forming the matrix, it is necessary to use polyacrylic acid and/or polyacrylamide to protect or compensate degradation of karaya during irradiation, if the matrix is to be sterilized. However, a predetermined concentration of salts, such as aluminum sulfate or sodium chloride, may be used in the matrix with karaya in some instances in lieu of polyacrylamide and/or polyacrylic acid. For example, concentrations of approximately 6% sodium chloride or aluminum sulfate may be used with karaya in forming the solid phase of the matrix.

It has been found that vinyl acetate dioctyl maleate copolymer may also be advantageously used in forming the solid phase of the matrix. Vinyl acetate dioctyl maleate copolymer (sold under the trademark "Flexbond 150" by Air Products and Chemicals Inc., and sold under the trademark "Bostik 8761" by the Bostik Company, Inc.) will intensify the tackiness of the bandage.

Another important gum material which may be used in forming the matrix is a starch graft copolymer sold under the trade name SGP 502S Absorbent Polymer by the Henkel Corporation, St. Paul, Minnesota. The starch graft copolymer product is derived from corn starch and acrylonitrile and is a graft terpolymer of starch, acrylamide and sodium acrylate. The technical name for this starch graft copolymer product is starch-g-poly® (acrylamide-co-sodium acrylate). The starch-g-poly material may be used alone to form the substrate or it may be used in combination with a synthetic gum such as acrylamide or a natural gum such

# EP 0 072 251 B1

as karaya. The starch-g-poly material is very effective as the skin contacting substrate since it does maintain its structural integrity and is non-toxic.

The following examples illustrate suitable substrate compositions for use in adhesive bandages according to the invention:—

### Example 1

|  | Nominal amounts of ingredients | Range of ingredients |
|---|---|---|
| Polyacrylamide | 5% | 1—50% |
| Karaya | 38% | 5—45% |
| Glycerol | 55% | 30—70% |
| Povidone-Iodine | 2% | 0.1—10% |

### Example 2

|  | Nominal amounts of ingredients | Range of ingredients |
|---|---|---|
| Polyacrylic acid | 10% | 2—40% |
| Polyacrylamide | 10% | 2—40% |
| Karaya | 18% | 5—45% |
| Glycerol | 60% | 30—70% |
| Povidone-Iodine | 2% | 0.1—10% |

### Example 3

|  | Nominal amounts of ingredients | Range of ingredients |
|---|---|---|
| Polyacrylamide | 15% | 2—40% |
| Polyacrylic acid | 15% | 2—40% |
| Glycerol | 68% | 30—70% |
| Povidone-Iodine | 2% | 0.1—10% |

### Example 4

|  | Nominal amounts of ingredients | Range of ingredients |
|---|---|---|
| Polyacrylamide | 30% | 2—40% |
| Glycerol | 62% | 50—70% |
| Methyl salicylate | 8% | 0.1—15% |

### Example 5

|  | Nominal amounts of ingredients | Range of ingredients |
|---|---|---|
| Polyacrylamide | 21.5% | 2—40% |
| Polyacrylic acid | 12.5% | 2—40% |
| Glycerol | 42% | 30—70% |
| Vinyl acetate-dioctyl maleate | 16% | 10—20% |
| Methyl salicylate | 8% | 0.1—15% |

### Example 6

|  | Nominal amounts of ingredients | Range of ingredients |
|---|---|---|
| Polyacrylamide | 32% | 2—40% |
| Glycerol | 55% | 30—70% |
| Water | 6% | 1—10% |
| Methyl salicylate | 8% | 0.1—15% |

### Example 7

|  | Nominal amounts of ingredients | Range of ingredients |
|---|---|---|
| Povidone-Iodine | 2% | 0.1—10% |
| Hydroxy-propylcellulose (Klucel®) | 6% | 0.1—10% |
| Glycerin | 56% | 30—70% |
| water | 6% | 0.1—10% |
| Polyacrylamide (Reten 421®) | 30% | 2—40% |

4

## Example 8

|  | Nominal amounts of ingredients | Range of ingredients |
|---|---|---|
| Povidone-Iodine | 10% | 0.1—15% |
| Reten 421® (polyacrylamide) | 5% | 2—40% |
| Karaya | 35% | 5—45% |
| Glycerol | 50% | 30—70% |

## Example 9

| Povidone-Iodine | 2% | 0.1—10% |
|---|---|---|
| Karaya | 42% | 5—45% |
| Glycerol | 55% | 30—70% |

## Example 10

| Camphor | 2% | 0.1—5% |
|---|---|---|
| Methylenebisacrylamide | 3% | 0.1—10% |
| Acrylic acid | 8% | 0.1—10% |
| Glycerol | 86% | 45—90% |
| Activators* | 1% | 0.1—2% |

*Potassium persulfate 0.6%
Sodium metabisulfite 0.2%
Ferrous sulfate 0.1%

## Example 11

| Camphor | 2% | 0.1—5% |
|---|---|---|
| Glycerol | 5% | 30—70% |
| Karaya | 43% | 5—45% |

## Example 12

| Methyl salicylate | 2% | 0.1—10% |
|---|---|---|
| Methylene bisacrylamide | 5% | 0.1—10% |
| Acrylic acid | 8% | 0.1—10% |
| Glycerol | 84% | 30—70% |
| Activators | 1% | 0.1—2% |

## Example 13

| Methyl salicylate | 8% | 0.1—15% |
|---|---|---|
| Acrylic acid | 2% | 0.1—10% |
| Methylenebisacrylamide | 1% | 0.1—10% |
| Glycerol | 48% | 30—70% |
| Karaya | 40% | 5—45% |
| Activators | 1% | 0.1—2% |

## Example 14

| Methyl salicylate | 8% | 0.1—15% |
|---|---|---|
| Karaya | 45% | 5—45% |
| Glycerol | 47% | 30—70% |

## Example 15

| Vinyl acetate dioctyl maleate copolymer | 62% | 50—75% |
|---|---|---|
| Vinyl acetate ethylene | 29% | 25—50% |
| NaCl | 1% | 1—5% |
| Methyl salicylate | 8% | 0.1—15% |

Example 16

| | Nominal amounts of ingredients | Range of ingredients |
|---|---|---|
| Vinyl acetate dioctyl maleate | 84% | 80—98% |
| Karaya | 12% | 5—45% |
| NaCl | 2% | 0.5—5% |
| Povidone-Iodine | 2% | 0.1—10% |

Example 17

| | Nominal amounts of ingredients | Range of ingredients |
|---|---|---|
| Starch-g-poly® | 30% | 15—50% |
| Glycerol | 62% | 30—70% |
| Nitroglycerine | 8% | 0.1—15% |

Example 18

| | Nominal amounts of ingredients | Range of ingredients |
|---|---|---|
| Starch-g-poly® | 25% | 1—30% |
| Glycerol | 55% | 30—70% |
| Karaya | 12% | 5—45% |
| Nitroglycerine | 8% | 0.1—15% |

**Claims**

1. A flexible adhesive bandage for delivering a medicament to a patient, which bandage comprises a flexible backing element and a substrate, which substrate contains a medicament, the bandage having an adhesive surface for contact with and adhesion to a patient's skin, characterised in that the backing element is selected from cotton, paper, synthetic fabrics, and plastics materials, the substrate is liquid-absorbent and self-adhesive and comprises a homogeneous, hydrophilic, stable matrix sufficiently pliant to conform to the shape of the body contours, the matrix comprising from 15% to 50% by weight, based on the total weight of the matrix, of a solid phase formed from a hydrophilic high-molecular weight polysaccharide and/or synthetic gum selected from polyacrylic acid, polyacrylamide, and their cogeners, vinyl acetate ethylene copolymer, vinyl acetate dioctyl maleate copolymer, natural gums, and acrylamide-co-sodium acrylate, and from 30% to 70% by weight, based on the total weight of the matrix, of a liquid phase consisting of a solution or emulsion selected from carbohydrates, proteins, and polyhydric alcohols, the matrix containing a medicament uniformly molecularly dispersed throughout the substrate, which medicament is selected from vaso dilators, antibacterial agents, antiseptic agents, antifungal agents, antihistamine agents, anti-inflammatory agents, antipruretic agents, hormonal agents, keratolytic agents, skin protective agents, and rubefacient agents.

2. A bandage as claimed in Claim 1, wherein the liquid phase comprises a polyhydric alcohol, such as glycerol.

3. A bandage as claimed in Claim 1 or Claim 2, wherein the solid phase of the matrix includes (a) a natural gum selected from gum karaya, gum acacia, locust bean gum, and guar gum and (b) polyacrylic acid and/or polyacrylamide, and the liquid phase comprises glycerol.

4. A bandage as claimed in any one of Claims 1 to 3, wherein the solid phase of the matrix includes a natural gum selected from gum karaya, gum acacia, locust bean gum, and guar gum.

5. A bandage as claimed in Claim 4, wherein the matrix comprises 5% to 45% by weight of gum karaya, 2% to 40% by weight of polyacrylamide, and 30% to 70% by weight of glycerol.

6. A bandage as claimed in any one of Claims 1 to 3 wherein the matrix comprises 10% to 50% by weight of polyacrylamide and 30% to 70% by weight of glycerol.

7. A bandage as claimed in any one of Claims 1 to 3, wherein the matrix is formed of 2% to 40% by weight of polyacrylamide and 2% to 40% by weight of polyacrylic acid, and 30% to 70% by weight of glycerol.

8. A bandage as claimed in any one of Claims 1 to 3 wherein the matrix is formed of 2% to 50% by weight of polyacrylic acid and 30% to 70% by weight of glycerol.

9. A bandage as claimed in any one of Claims 1 to 8, wherein the medicament comprises 0.1% to 15% by weight of povidone-iodine.

10. A bandage as claimed in any one of Claims 1 to 8, wherein the medicament comprises 0.1% to 5% by weight of camphor.

11. A bandage as claimed in any one of Claims 1 to 8, wherein the medicament comprises 0.1% to 15% by weight of methyl salicylate.

12. A bandage as claimed in any one of Claims 1 to 11, wherein the backing element comprises a pressure-sensitive adhesive element and defines said adhesive surface (11a) being above the substrate (12a) which contact the patients skin.

**Patentansprüche**

1. Flexibler Klebeverband zur Verabreichung von Medikamenten an einen Patienten mit einem flexiblen Trägerelement und einem das Medikament enthaltenden Substrat, wobei der Verband eine Haftoberfläche aufweist, die einen Haftkontakt zwischen Verband und der Haut des Patienten gewährleistet, dadurch gekennzeichnet, daß das Trägerelement aus Baumwolle, Papier, Synthetik oder Kunststoff besteht, daß das Substrat flüssigkeitsabsorbierend und selbstklebend ist und eine homogene, hydrophile und stabile Matrix aufweist, die ausreichend biegsam ist, um sich den Körperformen des Patienten anzupassen, wobei die Matrix bezogen auf ihr Gesamtgewicht zu 15—50 Gewichtsprozent aus einer festen Phase aufgebaut ist, die von einem hydrophilen und ein großes Molekulargewicht aufweisenden Polysaccharid und/oder einem synthetischen Gummi wie Polyacrylsäure, Polyacrylamid oder deren Verbindungen, Vinylacetat-Ethylen-Copolymer, Vinylacetat-Dioctylmaleat-Copolymer, natürlichen Gummen und Acrylamid-Co-Natrium-Acrylat gebildet ist, und zu 30—70 Gewichtsprozent, bezogen auf das Gesamtgewicht der Matrix, aus einer flüssigen Phase, die aus einer Lösung oder einer Emulsion von Kohlenhydraten, Proteinen oder Polyalkohlen gebildet ist, wobei die Matrix ein gleichförmig molekular über das gesamte Substrat verteiltes Medikament enthält, wie Mittel zur Gefäßerweiterung, Mittel gegen Juckreiz, antibakterielle Wirkstoffe, Antiseptika, Antimykotika, Antihistaminika, Antiphlogistika, hormonelle Wirkstoffe, Keratolytika, Hautschutzmittel und Rubefacientien.

2. Klebeverband nach Anspruch 1, dadurch gekennzeichnet, daß die flüssige Phase einen Polyalkohol wie Glycerol enthält.

3. Klebeverband nach Anspruch 1 oder 2, dadurch gekennzeichnet, daß die feste Phase der Matrix zum einen ein natürliches Gummi wie Karayagummi, Gummi arabicum, Johannisbrotkernmehl oder Guarmehl aufweist und zum anderen Polyacrylsäure und/oder Polyacrylamid aufweist, und daß die flüssige Phase Glycerol enthält.

4. Klebeverband nach einem der Ansprüche 1 bis 3, dadurch gekennzeichnet, daß die feste Phase der Matrix ein natürliches Gummi aufweist wie Karayagummi, Gummi arabicum, Johannisbrotkernmehl oder Guarmehl.

5. Klebeverband nach Anspruch 4, dadurch gekennzeichnet, daß die Matrix 5 bis 45 Gewichtsprozent Karayagummi, 2 bis 40 Gewichtsprozent Polyacrylamid und 30 bis 70 Gewichtsprozent Glycerol aufweist.

6. Klebeverband nach einem der Ansprüche 1 bis 3, dadurch gekennzeichnet, daß die Matrix 10 bis 50 Gewichtsprozent Polyacrylamid und 30 bis 70 Gewichtsprozent Glycerol aufweist.

7. Klebeverband nach einem der Ansprüche 1 bis 3, dadurch gekennzeichnet, daß die Matrix 2 bis 40 Gewichtsprozent Polyacrylamid und 2 bis 40 Gewichtsprozent Polyacrylsäure sowie 30 bis 70 Gewichtsprozent Glycerol aufweist.

8. Klebeverband nach einem der Ansprüche 1 bis 3, dadurch gekennzeichnet, daß die Matrix 2 bis 50 Gewichtsprozent Polyacrylsäure und 30 bis 70 Gewichtsprozent Glycerol aufweist.

9. Klebeverband nach einem der Ansprüche 1 bis 8, dadurch gekennzeichnet, daß das Medikament 0,1 bis 15 Gewichtsprozent Povidon-Jod enthält.

10. Klebeverband nach einem der Ansprüche 1 bis 8, dadurch gekennzeichnet, daß das Medikament 0,1 bis 5 Gewichtsprozent Campher enthält.

11. Klebeverband nach einem der Ansprüche 1 bis 8, dadurch gekennzeichnet, daß das Medikament 0,1 bis 15 Gewichtsprozent Methylsalicylsäureester enthält.

12. Klebeverband nach einem der Ansprüche 1 bis 11, dadurch gekennzeichnet, daß das Trägerelement ein druckempfindliches Haftelement aufweist mit einer Haftoberfläche (11a), die das mit der Haut des Patienten in Verbindung stehende Substrat (12a) überdeckt.

**Revendications**

1. Pansement adhésif souple pour l'administration d'un médicament à un patient, lequel pansement comprend un élément de support souple et un substrat qui contient un médicament, le pansement ayant une surface adhésive pour être en contact avec la peau du patient et y adhérer, caractérisé par le fait que l'élément de support est choisi parmi les tissus de coton, de papier, synthétiques et de matières plastiques, le substrat absorbe les liquides et est auto-adhésif et comprend une matrice stable homogène et hydrophile suffisamment souple pour s'adapter à la forme des contours du corps, la matrice comprenant, pour 15 à 50% en poids par rapport au poids total de la matrice, une phase solide constituée par un polysaccharide hydrophile de poids moléculaire élevé et/ou une gomme synthétique, choisis dans le groupe comprenant l'acide polyacrylique et le polyacrylamide et leurs homologues, les copolymères d'éthylène vinyle acétate, les copolymères de maléate dioctyle vinyl acétate, les gommes naturelles et l'acrylate d'acrylamide cosodium, et, pour 30 à 70% en poids, par rapport au poids total de la matrice, une phase liquide constituée d'une solution ou d'une émulsion choisie dans le groupe des carbohydrates, protéines et alcools polyhydriques, la matrice contenant un médicament uniformément dispersé moléculairement dans tout le substrat, lequel médicament est choisi dans le groupe comprenant les vasodilatateurs, les agents anti-bactériens, les agents antiseptiques, les agents fongicides, les agents antihistaminiques, les agents antiinflammatoires, les agents anti-prurigineux, les agents hormonaux, les agents keratolytiques, les agents de protection cutanée et les agents rubéfiants.

2. Pansement selon la revendication 1, caractérisé par le fait que la phase liquide contient un alcool polyhydrique tel que le glycérol.

3. Pansement selon la revendication 1 ou 2, caractérisé par le fait que la phase solide de la matrice comprend: a) une gomme naturelle choisie dans le groupe comprenant les gommes de karaya, d'acacia, de caroubier, de guar et b) un acide polyacrylique et/ou un polyacrylamide, la phase liquide contenant du glycérol.

4. Pansement selon l'une quelconque des revendications 1 à 3, caractérisé par le fait que la phase solide de la matrice contient une gomme naturelle choisie dans le groupe comprenant les gommes de karaya, d'acacia, de caroubier et de guar.

5. Pansement selon la revendication 4, caractérisé par le fait que la matrice contient 4 à 45% en poids de gomme de karaya, 2 à 40% en poids de polyacrylamide et 30 à 70% en poids de glycérol.

6. Pansement selon l'une quelconque des revendications 1 à 3, caractérisé par le fait que la matrice contient 10 à 50% en poids de polyacrylamide et 30 à 70% en poids de glycérol.

7. Pansement selon l'une quelconque des revendications 1 à 3, caractérisé par le fait que la matrice est formée de 2 à 40% en poids de polyacrylamide, de 2 à 40% en poids d'acide polyacrylique et de 30 à 70% en poids de glycérol.

8. Pansement selon l'une quelconque des revendications 1 à 3, caractérisé par le fait que la matrice est formée de 2 à 50% en poids d'acide polyacrylique et de 30 à 70% en poids de glycérol.

9. Pansement selon l'une quelconque des revendications 1 à 8, caractérisé par le fait que le médicament contient 0,1 à 15% en poids de povidone-iode.

10. Pansement selon l'une quelconque des revendications 1 à 8, caractérisé en par le fait que le médicament contient 0,1 à 5% en poids de camphre.

11. Pansement selon l'une quelconque des revendications 1 à 8, caractérisé par le fait que le médicament contient 0,1 à 15% en poids de méthylsalicylate.

12. Pansement selon l'une quelconque des revendications 1 à 11, caractérisé par le fait que l'élément de support contient un élément adhésif sensible à la pression et définit ladite surface adhésive (11a) se trouvant au-dessus du substrat (12a) et qui est en contact avec la peau du patient.

Fig. 1

Fig. 2

Fig. 3

Fig. 4

Fig. 5

1